# EUROPEAN PATENT APPLICATION

(11) **EP 1 029 925 A1**
(43) Date of publication of application: **23.08.2000**
(21) Application number: 00102566.7
(22) Date of filing: 07.02.2000
(51) Int. Cl.: C12P 7/18, C12N 1/20, C12N 9/04

(54) **Method for producing xylitol**

(30) Priority: 09.02.1999 JP 3146499; 12.07.1999 JP 19762199
(71) Applicant: Ajinomoto Co., Ltd., Tokyo (JP)
(72) Inventor: Takenaka, Yasuhiro, c/o Ajinomoto Co., Inc., Kawasaki-shi, Kanagawa (JP); Tonouchi, Naoto, c/o Ajinomoto Co., Inc., Kawasaki-shi, Kanagawa (JP); Yokozeki, Kenzo, c/o Ajinomoto Co., Inc., Kawasaki-shi, Kanagawa (JP)
(74) Representative: Strehl Schübel-Hopf & Partner

(57) **Abstract**

D-Xylulose is efficiently converted into xylitol by allowing a microorganism which is transformed with a gene encoding xylitol dehydrogenase and has an ability to supply reducing power to act on D-xylulose to produce xylitol, and collecting the produced xylitol.

SEQUENCE LISTING
<110> AJINOMOTO CO., INC.

<120> Method for Producing Xylitol

<130> EPA-53317

<140>
<141> 2000-02-07

<150> JP 11-031464
<151> 1999-02-09

<150> JP 11-197621
<151> 1999-07-12

<160> 2

<170> PatentIn Ver. 2.0

<210> 1
<211> 26
<212> DNA
<213> Artificial Sequence

<220>
<223> Description of Artificial Sequence: primer
<400> 1
cgggaattcg atatcatttt aatgaa 26

<210> 2
<211> 29
<212> DNA
<213> Artificial Sequence

<220>
<223> Description of Artificial Sequence: primer

<400> 2

ggcggatccg cagtcaatac cggcataga 29

## Description

### Background of the Invention

### Field of the Invention

The present invention relates to a method for producing xylitol. Xylitol is useful in the field of food industry, pharmaceutical and the like.

### Related Art

The demand of xylitol which is a naturally occurring sugar alcohol is expected to increase in future. Xylitol is a promising low-calorie sweetener because it has lower calories and exhibits comparable sweetness compared with sucrose. In addition, because of its anti-dental caries property, it is utilized as a dental caries preventive sweetener. Furthermore, because xylitol does not elevate glucose level in blood, it is utilized for fluid therapy in the treatment of diabetes.

The current industrial production of xylitol mainly relies on hydrogenation of D-xylose as disclosed in U.S. Patent No. 4,008,825. D-Xylose used as a raw material is obtained by hydrolysis of plant materials such as wood, straws, corn cobs, oat hulls and other xylan-rich materials.

However, such D-xylose produced by hydrolysis of plant materials suffers a drawback that it is rather expensive, and it is arisen from high production cost. For example, the low yield of the hydrolysis treatment of plant materials leads to low purity of the produced D-xylose. Therefore, the acid used for the hydrolysis and the dyes must be removed by ion exchange treatment after the hydrolysis treatment, and the resulting D-xylose must be further crystallized to remove other hemicellulosic saccharides. In order to obtain D-xylose suitable for foodstuffs, further purification would be required. Such ion exchange treatment and crystallization treatment invite the increase of production cost.

Therefore, several methods for producing xylitol have been developed, which utilize readily available raw materials and generate little waste. For example, there have been developed methods for producing xylitol utilizing other pentitols as a starting material. One of such readily available pentitols is D-arabitol, and D-arabitol can be produced by using yeast (*Can. J. Microbiol*., *31*, 1985, 467-471; *J. Gen. Microbiol., 139*, 1993, 1047-54).

Then, several methods for producing xylitol that utilize D-arabitol as the raw material have been developed. *Applied Microbiology*., 18, 1031-1035 (1969) reported a method of producing D-arabitol from glucose by fermentation using *Debaryomyces hansenii* ATCC20121, converting the D-arabitol into D-xylulose using *Acetobacter suboxydans*, and further converting the D-xylulose into xylitol with the aid of the action of *Candida guilliermondii* var. soya.

However, this method takes 112 hours to obtain xylitol with a yield of 39% using 5.3% of arabitol, and hence there is still room for improvement in its yield and productivity.

EP 403 392A (Roquette Freres) and EP421 882A (Roquette Freres) disclose methods comprising producing D-arabitol by fermentation using an osmosis-resistant yeast, then converting D-arabitol into D-xylulose using a bacterium belonging to the genus *Acetobacter*, the genus *Gluconobacter*, or the genus *Klebsiella*, forming a mixture of xylose and D-xylulose from the D-xylulose by the action of glucose (xylose) isomerase, and converting the obtained mixture of xylose and D-xylulose into xylitol by hydrogenation. There is also disclosed the production of xylitol comprising preliminarily concentrating xylose in the mixture of xylose and D-xylulose and converting the xylose into xylitol by hydrogenation.

However, those methods for the production of xylitol mentioned above utilize D-arabitol produced by fermentation as a starting material, and convert it by multiple process steps. Therefore, the processes are complicated, and less satisfactory ones in view of process economy.

### Summary of the Invention

In order to solve the aforementioned problems, the inventors of the present invention already found microorganisms having an ability to directly convert D-arabitol into xylitol, and developed a method for producing xylitol comprising allowing these microorganisms to act on D-arabitol, and harvesting the produced xylitol (Japanese Patent Application No. 10-258962). And they analyzed these microorganisms, and elucidated that D-arabitol dehydrogenase activity and D-xylulose reductase (xylitol dehydrogenase) activity were involved in this conversion reaction. Furthermore, they found that xylitol could be stably produced with high yield by performing the reaction with addition of a carbon source or NADH (reduced type of nicotinamide adenine dinucleotide) to supply reducing power (Japanese Patent Application No. 10-258961).

An object of the present invention is to provide a method for efficiently performing the conversion of D-xylulose into xylitol utilizing the aforementioned findings.

As a result of the present inventors' studies devoted to achieve the aforementioned object, it was found that xylitol can efficiently be produced from D-xylulose by using a microorganism which has an enhanced xylitol dehydrogenase activity and an ability to supply reducing power, and thus the present invention has been accomplished.

That is, the present invention provides a method for producing xylitol comprising the steps of reacting a microorganism which is transformed by a gene encoding xylitol dehydrogenase and has an ability to supply reducing power with D-xylulose to produce xylitol, and collecting the produced xylitol.

According to a preferred embodiment of the aforementioned method of the present invention, the microorganism having an ability to supply reducing power is a bacterium belonging to the genus *Escherichia*. As such bacterium belonging to the genus *Escherichia*, *Escherichia coli* can be mentioned.

According to another preferred embodiment of the aforementioned method of the present invention, the method comprises steps of reacting a microorganism which has an ability to convert D-arabitol into D-xylulose with D-arabitol to produce D-xylulose, and reacting the produced D-xylulose with a microorganism which is transformed with a gene encoding xylitol dehydrogenase and has an ability to supply reducing power.

According to a further preferred embodiment of the aforementioned method of the present invention, the method comprises steps of culturing a microorganism which has an ability to produce D-xylulose from glucose in a suitable medium to produce D-xylulose, and reacting the produced D-xylulose with a microorganism which is transformed by a gene encoding xylitol dehydrogenase and has an ability to supply reducing power in the medium.

The present invention also provides a method for producing xylitol comprising steps of culturing a microorganism which is transformed with a gene encoding xylitol dehydrogenase and has an ability to supply reducing power and a microorganism which has an ability to convert D-arabitol into D-xylulose in a medium containing D-arabitol to produce xylitol, and collecting the produced xylitol.

The present invention further provides a method for producing xylitol comprising steps of culturing a microorganism which is transformed with a xylitol dehydrogenase and has an ability to supply reducing power and a microorganism which has an ability to produce D-xylulose from glucose in a suitable medium to produce xylitol, and collecting the produced xylitol.

### PREFERRED EMBODIMENTS OF THE INVENTION

Hereafter, the present invention will be explained in detail.

The microorganism used for the present invention is a microorganism transformed with a gene encoding xylitol dehydrogenase and having an ability to supply reducing power. A microorganism having such ability may be referred to as the "microorganism of the present invention".

The microorganism of the present invention can be obtained by transforming a microorganism having an ability to supply reducing power with a gene encoding xylitol dehydrogenase. The term "ability to supply reducing power" used for the present invention means an ability to supply NADH (reduced type of nicotinamide adenine dinucleotide) in an amount sufficient for allowing proceeding of the reaction for reducing D-xylulose to convert it into xylitol, which is catalyzed by xylitol dehydrogenase (D-xylulose reductase). As mentioned above, it has been suggested that the cause of the remaining of unreacted D-xylulose observed when xylitol is produced by using a microorganism having an ability to directly converting D-arabitol into xylitol is insufficient supply of NADH required for the reaction. And the inventors of the present invention have found that the reaction efficiently proceeded when a carbon source or NADH was added to the conversion reaction from D-arabitol to xylitol. In contrast, a microorganism with which the reduction reaction of D-xylulose sufficiently proceeds without adding a carbon source or NADH to a reaction system, when the microorganism is transformed with a gene encoding xylitol dehydrogenase, is the microorganism having an ability to supply reducing power referred to in the present invention. More specifically, a microorganism producing xylitol with a yield of 85% when it was allowed to act on D-xylulose at a concentration of 5% under suitable reaction conditions is a microorganism having an ability to supply reducing power of the present invention. Examples of such a microorganism include, for example, a bacterium belonging to *Escherichia*. As such a bacterium belonging to *Escherichia*, *Escherichia coli* can be mentioned.

Specifically, in order to transform a microorganism having an ability to supply reducing power with a gene encoding xylitol dehydrogenase, a recombinant DNA can be prepared by ligating a gene fragment encoding xylitol dehydrogenase to a vector functioning in the microorganism, preferably a multi-copy type vector, and introduced into the microorganism to transform it.

As the source of the gene encoding xylitol dehydrogenase, any microorganisms may be used so long as they have xylitol dehydrogenase. Examples of such microorganisms include, for example, *Gluconobacter cerinus*, *Gluconobacter oxydans*, *Acetobacter aceti*, *Acetobacter liquefaciens*, *Acetobacter pasteurianus*, *Frateuria aurantia*, *Bacillus subtilis*, *Bacillus* *megaterium*, *Proteus rettgeri*, *Serratia marcescens*, *Corynebacterium callunae*, *Brevibacterium ammoniagenes*, *Flavobacterium aurantinum*, *Flavobacterium rhenanum*, *Pseudomonas badiofaciens*, *Pseudomonas chlororaphis*, *Pseudomonas iners*, *Rhodococcus rhodochrous*, *Achromobacter viscosus*, *Agrobacterium tumefaciens*, *Agrobacterium radiobacter*, *Arthrobacter paraffineus*, *Arthrobacter hydrocarboglutamicus*, *Azotobacter indicus*, *Brevibacterium ketoglutamicum*, *Corynebacterium faciens*, *Erwinia amylovora*, *Flavobacterium peregrinum*, *Flavobacterium fucatum*, *Micrococcus* sp. CCM825, *Nocardia opaca*, *Planococcus eucinatus*, *Pseudomonas synxantha*, *Rhodococcus erythropolis*, *Morganella morganii*, *Actinomadura madurae*, *Actinomyces violaceochromogenes*, *Streptomyces coelicolor*, *Streptomyces flavelus*, *Streptomyces griseolus*, *Streptomyces lividans*, *Streptomyces olivaceus*, *Streptomyces tanashiensis*, *Streptomyces virginiae*, *Streptomyces antibioticus*, *Streptomyces cacaoi*, *Streptomyces lavendulae*, *Pichia stipitis* and so forth.

Among the aforementioned microorganisms, the nucleotide sequences of the genes encoding xylitol dehydrogenase derived from, for example, *Pichia stipitis* (*FEBS Lett*., *324*, 9 (1993)) and *Morganella morganii* (DDBJ/GenBank/EMBL Accession No. L34345) have been reported, and therefore the gene encoding xylitol dehydrogenase can be obtained by synthesizing primers based on the nucleotide sequences of these genes encoding xylitol dehydrogenase, and performing polymerase chain reaction (PCR: see White, T.J. *et al*., *Trends Genet*., *5*, 185 (1989)) using chromosome DNA of microorganisms such as *Morganella morganii* ATCC25829 as a template. Specific examples of the primers include the oligonucleotides for amplifying the gene encoding xylitol dehydrogenase of *Morganella morganii*, which have the nucleotide sequences represented in Sequence Listing as SEQ ID NOD: 1 and 2.

The vector used for introducing a gene encoding xylitol dehydrogenase into a host microorganism may be any vector so long as it can replicate in the host microorganism. Specific examples thereof include plasmid vectors such as pBR322, pTWV228, pMW119, pUC19 and pUC18.

To prepare a recombinant DNA containing the gene encoding xylitol dehydrogenase by ligating it to a vector which functions in *Escherichia* bacteria, the vector can be digested with a restriction enzyme matching the terminal sequence of the gene encoding xylitol dehydrogenase, and the both sequences can be ligated. The ligation is usually attained by using a ligase such as T4 DNA ligase.

The recombinant plasmid prepared as described above can be introduced into a host microorganism by a method reported for *Escherichia coli* such as a method of D. A. Morrison (Methods in Enzymology, 68, 326 (1979)) or a method in which recipient cells are treated with calcium chloride to increase permeability for DNA (Mandel, M. and Higa, A., *J. Mol. Biol*., *53*, 159 (1970)). Alternatively, the gene encoding xylitol dehydrogenase can also be incorporated into chromosome of the host by a method utilizing transduction, transposon (Berg, D.E. and Berg, C.M., *Bio/Technol*., *1*, 417 (1983)), Mu phage (Japanese Patent Laid-open [KOKAI] No. 2-109985), or homologous recombination (Experiments in Molecular Genetics, Cold Spring Harbor Lab. (1972)).

As the promoter for the expression of the gene encoding xylitol dehydrogenase, when a promoter specific for the gene encoding xylitol dehydrogenase functions in host cells, this promoter can be used. Alternatively, it is also possible to ligate a foreign promoter to a DNA encoding xylitol dehydrogenase so as to obtain the expression under the control of the promoter. As such a promoter, when an *Escherichia* bacterium is used as the host, *lac* promoter, *trp* promoter, *trc* promoter, *tac* promoter, P_{R} promoter and P_{L} promoter of lambda phage, *tet* promoter, *amyE* promoter, *spac* promoter and so forth can be used. Further, it is also possible to use an expression vector containing a promoter like pUC19, and insert a DNA fragment encoding xylitol dehydrogenase into the vector so that the fragment can be expressed under the control of the promoter.

When a microorganism contains a gene encoding xylitol dehydrogenase, xylitol dehydrogenase activity can be enhanced by replacing an expression regulatory sequence with a stronger one such as the promoter of the gene itself (see Japanese Patent Laid-open No. 1-215280). For example, all of the aforementioned promoters functioning in *Escherichia* bacteria have been known as strong promoters.

Methods for preparation of chromosome DNA, PCR, preparation of plasmid DNA, digestion and ligation of DNA, transformation, design and synthesis of oligonucleotides used as primers and so forth may be usual ones well known to those skilled in the art. Such methods are described in, for example, Sambrook, J., Fritsch, E.F., and Maniatis, T., "Molecular Cloning: A Laboratory Manual, Second Edition", Cold Spring Harbor Laboratory Press (1989) and so forth.

Xylitol can be produced by allowing a microorganism transformed with a gene encoding xylitol dehydrogenase obtained as described above and having an ability to supply reducing power to act on D-xylulose, and collecting the produced xylitol.

The medium used for the culture of the microorganism transformed with a gene encoding xylitol dehydrogenase may be a usual medium which contains a carbon source, nitrogen source, inorganic ions suitable for the microorganism, as well as other organic components, if necessary.

As the carbon source, it is possible to use sugars such as glucose, lactose, galactose, fructose or starch hydrolysate; alcohols such as glycerol or sorbitol; or organic acids such as fumaric acid, citric acid or succinic acid.

As the nitrogen source, it is possible to use inorganic ammonium salts such as ammonium sulfate, ammonium chloride or ammonium phosphate; organic nitrogen such as soybean hydrolysate; ammonia gas; or aqueous ammonia.

It is desirable to allow required substances such as vitamins or yeast extract to be contained in appropriate amounts as organic trace nutrients. Other than the above, potassium phosphate, magnesium sulfate, iron ion, manganese ion and the like are added in small amounts, if necessary.

Cultivation is preferably carried out under an aerobic condition for 16-120 hours. The cultivation temperature is preferably controlled at 25°C to 45°C, and pH is preferably controlled at 5-8 during cultivation. Inorganic or organic, acidic or alkaline substances as well as ammonia gas or the like can be used for pH adjustment.

Xylitol can be produced in a reaction mixture by contacting a culture containing cells cultured as described above, cells separated and collected from the culture, processed cells subjected to acetone treatment or lyophillization, cell free extract prepared from such cells or processed cells, fractions such as membrane fractions fractionated from such cell free extract, or immobilized materials produced by immobilizing these cells, processed cells, cell free extract and fractions with D-xylulose, and allowing the reaction. The microorganism may consist of one kind of microorganism, or used as an arbitrary mixture of two or more kinds of microorganisms.

By culturing the microorganism of the present invention in a medium containing D-xylulose, xylitol can also be produced in the medium. While the D-xylulose may be produced by any methods, D-xylulose produced by allowing a microorganism having an ability to convert D-arabitol into D-xylulose to act on D-arabitol, for example, can preferably be used for the present invention. Moreover, D-xylulose produced by using a microorganism having an ability to produce D-xylulose from glucose can also be used for the present invention.

If glucose or glycerol is added to a reaction mixture or medium, which is used when the microorganism of the present invention or a processed material obtained from it is allowed to act on D-xylulose, efficiency of the conversion of D-xylulose into xylitol may be improved.

For example, xylitol can also be produced from D-arabitol by culturing a microorganism having an ability to convert D-arabitol into D-xylulose in a medium containing D-arabitol, and then culturing the microorganism of the present invention using the same medium. Moreover, xylitol can also be produced from glucose by culturing a microorganism having an ability to produce D-xylulose from glucose in a suitable medium, and then culturing the microorganism of the present invention using the same medium. Furthermore, xylitol can also be produced by culturing the microorganism of the present invention in a suitable medium together with a microorganism having an ability to convert D-arabitol into D-xylulose or a microorganism having an ability to produce D-xylulose from glucose.

Examples of the microorganism having an ability to convert D-arabitol into D-xylulose include, for example, microorganisms belonging to the genus *Gluconobacter*, *Achromobacter*, *Agrobacterium*, *Alcaligenes*, *Arthrobacter*, *Azotobacter*, *Brevibacterium*, *Corynebacterium*, *Enterobacter*, *Erwinia*, *Flavobacterium*, *Micrococcus*, *Morganella*, *Nocardia*, *Planococcus*, *Proteus*, *Propionibacterium*, *Pseudomonas*, *Rhodococcus*, *Sporosarcina*, *Staphylococcus*, *Vibrio*, *Actinomadura*, *Actinomyces*, *Kitasatosporia*, *Streptomyces*, *Aeromonas*, *Aureobacterium*, *Bacillus*, *Escherichia*, *Microbacterium*, *Serratia*, *Salmonella* or *Xanthomonas*.

More specifically, examples of the aforementioned microorganisms include *Gluconobacter oxydans*, *Gluconobacter asaii*, *Achromobacter delmarvae*, *Achromobacter viscosus*, *Achromobacter lacticum*, *Agrobacterium tumefaciens*, *Agrobacterium radiobacter*, *Alcaligenes faecalis*, *Arthrobacter citreus*, *Arthrobacter tumescens*, *Arthrobacter paraffineus*, *Arthrobacter hydrocarboglutamicus*, *Arthrobacter oxydans*, *Aureobacterium saperdae*, *Azotobacter indicus*, *Brevibacterium ammoniagenes*, *divaricatum*, *Brevibacterium lactofermentum*, *Brevibacterium flavum*, *Brevibacterium globosum*, *Brevibacterium fuscum*, *Brevibacterium ketoglutamicum*, *Brevibacterium helcolum*, *Brevibacterium pusillum*, *Brevibacterium testaceum*, *Brevibacterium roseum*, *Brevibacterium immariophilium*, *Brevibacterium linens*, *Brevibacterium protopharmiae*, *Corynebacterium acetophilum*, *Corynebacterium glutamicum*, *Corynebacterium callunae*, *Corynebacterium acetoacidophilum*, *Corynebacterium acetoglutamicum*, *Enterobacter aerogenes*, *Erwinia amylovora*, *Erwinia carotovora*, *Erwinia herbicola*, *Erwinia chrysanthemi*, *Flavobacterium peregrinum*, *Flavobacterium fucatum*, *Flavobacterium aurantinum*, *Flavobacterium rhenanum*, *Flavobacterium sewanense*, *Flavobacterium breve*, *Flavobacterium meningosepticum*, *Micrococcus sp*. *CCM825*, *Morganella morganii*, *Nocardia opaca*, *Nocardia rugosa*, *Planococcus eucinatus*, *Proteus rettgeri*, *Propionibacterium shermanii*, *Pseudomonas synxantha*, *Pseudomonas azotoformans*, *Pseudomonas* *fluorescens*, *Pseudomonas ovails*, *Pseudomonas stutzeri*, *Pseudomonas acidovolans*, *Pseudomonas mucidolens*, *Pseudomonas testosteroni*, *Pseudomonas aeruginosa*, *Rhodococcus erythropolis*, *Rhodococcus rhodochrous*, *Rhodococcus sp. ATCC 15592*, *Rhodococcus sp. ATCC 19070*, *Sporosarcina ureae*, *Staphylococcus aureus*, *Vibrio metschnikovii*, *Vibrio tyrogenes*, *Actinomadura madurae*, *Actinomyces violaceochromogenes*, *Kitasatosporia parulosa*, *Streptomyces coelicolor*, *Streptomyces flavelus*, *Streptomyces griseolus*, *Streptomyces lividans*, *Streptomyces olivaceus*, *Streptomyces tanashiensis*, *Streptomyces virginiae*, *Streptomyces antibioticus*, *Streptomyces cacaoi*, *Streptomyces lavendulae*, *Streptomyces viridochromogenes*, *Aeromonas salmonicida*, *Bacillus pumilus*, *Bacillus circulans*, *Bacillus thiaminolyticus*, *Escherichia coli*, *Escherichia freundii*, *Microbacterium ammoniaphilum*, *Serratia marcescens*, *Salmonella typhimurium*, *Salmonella schottmulleri*, *Xanthomonas citri* and so forth.

Furthermore, the following strains are mentioned as specific bacterial strains.
*Gluconobacter oxydans* ATCC621
*Gluconobacter suboxydans* NRRL B-755
*Gluconobacter oxydans* subsp. oxydans IFO14819
*Gluconobacter oxydans* IAM 1842
*Gluconobacter asaii* IFO3276
*Achromobacter delmarvae* AJ1983
*Achromobacter viscosus* ATCC12448
*Achromobacter lacticum* AJ2394
*Agrobacterium tumefaciens* ATCC4720
*Alcaligenes faecalis* IAM1015
*Arthrobacter citreus* ATCC11624
*Arthrobacter tumescens* ATCC6947
*Arthrobacter paraffineus* ATCC15590
*Arthrobacter paraffineus* ATCC15591
*Arthrobacter paraffineus* ATCC19064
*Arthrobacter paraffineus* ATCC19065
*Arthrobacter hydrocarboglutamicus* ATCC15583
*Arthrobacter oxydans* ATCC 14358
*Azotobacter indicus* ATCC9037
*Brevibacterium divaricatum* ATCC14020
*Brevibacterium lactofermentum* ATCC13655
*Brevibacterium lactofermentum* ATCC13869
*Brevibacterium flavum* ATCC13826
*Brevibacterium flavum* ATCC21406
*Brevibacterium globosum* AJ1563
*Brevibacterium fuscum* AJ3124
*Brevibacterium ketoglutamicum* ATCC15587
*Brevibacterium ketoglutamicum* ATCC15588
*Brevibacterium helcolum* ATCC11822
*Brevibacterium pusillum* ATCC19096
*Brevibacterium testaceum* AJ1464
*Brevibacterium roseum* ATCC13825
*Brevibacterium immariophilium* ATCC14068
*Brevibacterium linens* ATCC8377
*Brevibacterium protopharmiae* AJ3125
*Corynebacterium acetophilum* NRRLB3421
*Corynebacterium glutamicum* ATCC 13059
*Corynebacterium callunae* ATCC 15991
*Corynebacterium acetoacidophilum* ATCC 13870
*Corynebacterium acetoacidophilum* ATCC 21407
*Corynebacterium acetoglutamicum* ATCC15806
*Enterobacter aerogenes* ATCC13048
*Erwinia amylovora* IFO12687
*Erwinia carotovora* CCM969
*Erwinia carotovora* AJ2992
*Erwinia herbicola* ATCC 23822
*Erwinia chrysanthemi* ATCC 11662
*Flavobacterium peregrinum* CCM1080-A
*Flavobacterium fucatum* AJ2478
*Flavobacterium aurantinum* AJ2466
*Flavobacterium rhenanum* AJ2468
*Flavobacterium sewanense* AJ2476
*Flavobacterium breve* ATCC 14234
*Flavobacterium meningosepticum* ATCC 13253
*Micrococcus* sp. CCM825
*Nocardia opaca* NCIB9409
*Nocardia rugosa* NCIB 8926
*Planacoccus eucinatus* AJ1656
*Proteus rettgeri* NRRL B-11344
*Proteus rettgeri* AJ2769
*Proteus rettgeri* AJ2770
*Morganella morganii* AJ2771
*Propionibacterium shermanii* IAM1725
*Pseudomonas synxantha* ATCC796
*Pseudomonas azotoformans* IFO 12693
*Pseudomonas fluorescens* IFO 3757
*Pseudomonas fluorescens* ATCC 13525
*Pseudomonas ovalis* IAM 1201
*Pseudomonas stutzeri* IAM 1504
*Pseudomonas acidovolans* ATCC 9355
*Pseudomonas mucidolens* ATCC4685
*Pseudomonas testosteroni* ATCC17409
*Pseudomonas aeruginosa* ATCC15528
*Rhodococcus erythropolis* ATCC11048
*Rhodococcus rhodochrous* ATCC 4273
*Rhodococcus rhodochrous* ATCC 21199
*Rhodococcus rhodochrous* ATCC 21198
*Rhodococcus rhodochrous* ATCC 19149
*Rhodococcus rhodochrous* ATCC 21197
*Rhodococcus* sp. ATCC 15592
*Rhodococcus* sp. ATCC 19070
*Sporosarcina ureae* AJ1232
*Staphylococcus aureus* IFO3761
*Vibrio metschnikovii* ATCC7708
*Vibrio tyrogenes* AJ2807
*Actinomadura madurae* ATCC19425
*Actinomyces violaceochromogenes* IFO13100
*Kitasatosporia parulosa* AJ9458
*Streptomyces coelicolor* ATCC10147
*Streptomyces flavelus* AJ9012
*Streptomyces griseolus* NRRL B-1062
*Streptomyces lividans* IFO13385
*Streptomyces olivaceus* ATCC21379
*Streptomyces tanashiensis* ATCC15238
*Streptomyces virginiae* AJ9053
*Streptomyces antibioticus* NRRL3238
*Streptomyces cacaoi* ATCC19093
*Streptomyces lavendulae* ATCC8664
*Streptomyces viridochromogenes* IFO3113
*Aeromonas salmonicida* ATCC 14174
*Aureobacterium saperdae* ATCC19272
*Bacillus pumilus* IAM 1244
*Bacillus circulans* ATCC 9966
*Bacillus thiaminolyticus* IAM 103
*Escherichia coli* ATCC10798
*Escherichia coli* IAM 1204
*Escherichia coli* IAM 1239
*Escherichia coli* IAM 1518
*Escherichia coli* IAM 1519
*Escherichia coli* IAM 1137
*Escherichia freundii* IFM S-36
*Microbacterium ammoniaphilum* ATCC 15354
*Serratia marcescens* CCM958
*Salmonella typhimurium* AJ2636
*Salmonella schottmulleri* ATCC 8759
*Xanthomonas citri* IAM1648

*Achromobacter lacticum* AJ2394 was deposited at the Fermentation Research Institute, Agency of Industrial Science and Technology, Ministry of International Trade and Industry (presently the National Institute of Bioscience and Human-Technology, Agency of Industrial Science and Technology, Ministry of International Trade and Industry) on January 20, 1984, and received an accession number of FERM P-7401. This strain was transferred to the international deposit on January 6, 2000, and received an accession number of FERM BP-6981.

*Brevibacterium testaceum* AJ1464 was deposited at the Fermentation Research Institute, Agency of Industrial Science and Technology, Ministry of International Trade and Industry (presently the National Institute of Bioscience and Human-Technology, Agency of Industrial Science and Technology, Ministry of International Trade and Industry) on July 11, 1987, and received an accession number of FERM P-9469. This strain was transferred to the international deposit on January 6, 2000, and received an accession number of FERM BP-6984.

*Brevibacterium protopharmiae* AJ3125 was deposited at the Fermentation Research Institute, Agency of Industrial Science and Technology, Ministry of International Trade and Industry (presently the National Institute of Bioscience and Human-Technology, Agency of Industrial Science and Technology, Ministry of International Trade and Industry) on February 23, 1995, and received an accession number of FERM P-14784. This strain was transferred to the international deposit on January 6, 2000, and received an accession number of FERM BP-6985.

*Erwinia carotovora* AJ2992 was deposited at the Fermentation Research Institute, Agency of Industrial Science and Technology, Ministry of International Trade and Industry (presently the National Institute of Bioscience and Human-Technology, Agency of Industrial Science and Technology, Ministry of International Trade and Industry) on January 19, 1987, and received an accession number of FERM P-9135. This strain was transferred to the international deposit on October 27, 1987, and received an accession number of FERM BP-1538.

*Flavobacterium aurantinum* AJ2466 was deposited at the Fermentation Research Institute, Agency of Industrial Science and Technology, Ministry of International Trade and Industry (presently the National Institute of Bioscience and Human-Technology, Agency of Industrial Science and Technology, Ministry of International Trade and Industry) on January 20, 1984, and received an accession number of FERM P-7402. This strain was transferred to the international deposit on January 6, 2000, and received an accession number of FERM BP-6982.

*Flavobacterium rhenanum* AJ2468 was deposited at the Fermentation Research Institute, Agency of Industrial Science and Technology, Ministry of International Trade and Industry (presently the National Institute of Bioscience and Human-Technology, Agency of Industrial Science and Technology, Ministry of International Trade and Industry) on September 30, 1985, and received an accession number of FERM P-8459. This strain was transferred to the international deposit on April 21, 1988, and received an accession number of FERM BP-1862.

*Flavobacterium sewanense* AJ2476 was deposited at the Fermentation Research Institute, Agency of Industrial Science and Technology, Ministry of International Trade and Industry (presently the National Institute of Bioscience and Human-Technology, Agency of Industrial Science and Technology, Ministry of International Trade and Industry) on April 25, 1983, and received an accession number of FERM P-7052. This strain was transferred to the international deposit on February 2, 1984, and received an accession number of FERM BP-476.

*Salmonella typhimurium* AJ2636 was deposited at the Fermentation Research Institute, Agency of Industrial Science and Technology, Ministry of International Trade and Industry (presently the National Institute of Bioscience and Human-Technology, Agency of Industrial Science and Technology, Ministry of International Trade and Industry) on July 11, 1987, and received an accession number of FERM P-9470. This strain was transferred to the international deposit on November 2, 1998, and received an accession number of FERM BP-6566.

*Proteus rettgeri* NRRL B-11344 was deposited at the Agricultural Research Service Culture Collection on July 13, 1978 as the international deposit, and received an accession number of NRRL B-11344.

*Proteus rettgeri* AJ2770 was deposited at the Fermentation Research Institute, Agency of Industrial Science and Technology, Ministry of International Trade and Industry (presently the National Institute of Bioscience and Human-Technology, Agency of Industrial Science and Technology, Ministry of International Trade and Industry) on November 28, 1985 as the international deposit, and received an accession number of FERM BP-941.

*Sporosarcina ureae* AJ1232 was deposited at the Fermentation Research Institute, Agency of Industrial Science and Technology, Ministry of International Trade and Industry (presently the National Institute of Bioscience and Human-Technology, Agency of Industrial Science and Technology, Ministry of International Trade and Industry) on April 25, 1983, and received an accession number of FERM P-7050. This strain was transferred to the international deposit on January 6, 2000, and received an accession number of FERM BP-6979.

*Flavobacterium fucatum* AJ2478 was deposited at the Fermentation Research Institute, Agency of Industrial Science and Technology, Ministry of International Trade and Industry (presently the National Institute of Bioscience and Human-Technology, Agency of Industrial Science and Technology, Ministry of International Trade and Industry) on April 25, 1983, and received an accession number of FERM P-7053. This strain was transferred to the international deposit on September 9, 1998, and received an accession number of FERM BP-6492.

*Planococcus eucinatus* AJ1656 was deposited at the Fermentation Research Institute, Agency of Industrial Science and Technology, Ministry of International Trade and Industry (presently the National Institute of Bioscience and Human-Technology, Agency of Industrial Science and Technology, Ministry of International Trade and Industry) on January 19, 1987, and received an accession number of FERM P-9133. This strain was transferred to the international deposit on September 9, 1998, and received an accession number of FERM BP-6493.

*Proteus rettgeri* AJ2769 was deposited at the Fermentation Research Institute, Agency of Industrial Science and Technology, Ministry of International Trade and Industry (presently the National Institute of Bioscience and Human-Technology, Agency of Industrial Science and Technology, Ministry of International Trade and Industry) on April 25, 1983, and received an accession number of FERM P-7057. This strain was transferred to the international deposit on January 6, 2000, and received an accession number of FERM BP-6980.

*Vibrio tyragenes* AJ2807 was deposited at the Fermentation Research Institute, Agency of Industrial Science and Technology, Ministry of International Trade and Industry (presently the National Institute of Bioscience and Human-Technology, Agency of Industrial Science and Technology, Ministry of International Trade and Industry) on April 25, 1983, and received an accession number of FERM P-7060. This strain was transferred to the international deposit on March 4, 1997, and received an accession number of FERM BP-5848.

*Achromobacter delmarvae* AJ1983 was deposited at the Fermentation Research Institute, Agency of Industrial Science and Technology, Ministry of International Trade and Industry (presently the National Institute of Bioscience and Human-Technology, Agency of Industrial Science and Technology, Ministry of International Trade and Industry) on January 16, 1998, and received an accession number of FERM P-16593. This strain was transferred to the international deposit on January 6, 2000, and received an accession number of FERM BP-6988

*Brevibacterium globosum* AJ1563 was deposited at the Fermentation Research Institute, Agency of Industrial Science and Technology, Ministry of International Trade and Industry (presently the National Institute of Bioscience and Human-Technology, Agency of Industrial Science and Technology, Ministry of International Trade and Industry) on January 16, 1998, and received an accession number of FERM P-16590. This strain was transferred to the international deposit on January 6, 2000, and received an accession number of FERM BP-6987.

*Brevibacterium fuscum* AJ3124 was deposited at the Fermentation Research Institute, Agency of Industrial Science and Technology, Ministry of International Trade and Industry (presently the National Institute of Bioscience and Human-Technology, Agency of Industrial Science and Technology, Ministry of International Trade and Industry) on April 23, 1985, and received an accession number of FERM P-8194. This strain was transferred to the international deposit on January 6, 2000, and received an accession number of FERM BP-6983.

*Morganella morganii* AJ2771 was deposited at the Fermentation Research Institute, Agency of Industrial Science and Technology, Ministry of International Trade and Industry (presently the National Institute of Bioscience and Human-Technology, Agency of Industrial Science and Technology, Ministry of International Trade and Industry) on January 16, 1998, and received an accession number of FERM P-16594. This strain was transferred to the international deposit on September 9, 1998, and received an accession number of FERM BP-6496.

*Kitasatosporia parulosa* AJ9458 was deposited at the Fermentation Research Institute, Agency of Industrial Science and Technology, Ministry of International Trade and Industry (presently the National Institute of Bioscience and Human-Technology, Agency of Industrial Science and Technology, Ministry of International Trade and Industry) on January 16, 1998, and received an accession number of FERM P-16588. This strain was transferred to the international deposit on January 6, 2000, and received an accession number of FERM BP-6986.

*Streptomyces flavelus* AJ9012 was deposited at the Fermentation Research Institute, Agency of Industrial Science and Technology, Ministry of International Trade and Industry (presently the National Institute of Bioscience and Human-Technology, Agency of Industrial Science and Technology, Ministry of International Trade and Industry) on January 16, 1998, and received an accession number of FERM P-16585. This strain was transferred to the international deposit on September 9, 1998, and received an accession number of FERM BP-6494.

*Streptomyces virginiae* AJ9053 was deposited at the Fermentation Research Institute, Agency of Industrial Science and Technology, Ministry of International Trade and Industry (presently the National Institute of Bioscience and Human-Technology, Agency of Industrial Science and Technology, Ministry of International Trade and Industry) on January 16, 1998, and received an accession number of FERM P-16587. This strain was transferred to the international deposit on September 9, 1998, and received an accession number of FERM BP-6495.

*Erwinia carotovora* CCM969, *Flavobacterium peregrinum* CCM1080-A, *Micrococcus* sp. CCM825 and *Serratia marcescens* CCM958 are available from the Czechoslovak Collection of Microorganisms (Address: Tvrdeho 14, Brno CS-602 00, Czechoslovakia).

*Nacardia opaca* NCIB9409 and *Nocardia rugosa* NCIB 8926 are available from the National Collections of Industrial and Marine Bacteria (Address: NCIMB Lts., Torry Research Station 135 Abbey Road, Aberdeen AB9 8DG, United Kingdom).

*Gluconobacter oxydans* subsp. oxydans IFO14819, *Gluconobacter asaii* IFO3276, *Staphylococcus aureus* IFO3761, *Erwinia amylovora* IFO12687, *Actinomyces violaceochromogenes* IFO13100, *Streptomyces lividans* IFO13385 and *Streptomyces viridochromogenes* IFO3113 are available from the Institute for Fermentation, Osaka (Address: 17-85, Juso-honmachi 2-chome, Yodogawa-ku, Osaka 532, Japan).

*Gluconobacter oxydans* IAM 1842, *Propionibacterium shermanii* IAM1725, *Alcaligenes faecalis* IAM1015, IAM1725, *Bacillus pumlius* IAM 1244, *Escherichia coli* IAM 1204, *Escherichia coli* IAM 1239, *Escherichia coli* IAM 1518, *Escherichia coli* IAM 1519, *Escherichia coli* IAM 1137, and *Xanthomonas citri* IAM1648 are available from the Institute of Molecular and Cellular Biosciences (Formerly, Institute of Applied Microbiology, Address: The university of Tokyo, Yayoi 1-chome, Bunkyo-ku, Tokyo, Japan).

*Escherichia freundii* IFM S-36 are available from the Research Center for Pathogenic Funji and Microbial Toxicoses, Chiba University (Address: 8-1, Inohara 1-chomme, Chuo-ku, Chiba-Shi, Chiba, Japan).

The other strains are available from the American Type Culture Collection (Address: 12301 Parklawn Drive, Rockville, Maryland 20852, United States of America).

The medium used for culturing these microorganisms is not particularly limited, and it may be an ordinary medium containing a carbon source, nitrogen source and inorganic ions, as well as organic nutrients, if necessary. As the carbon source, carbohydrates such as glucose, alcohols such as glycerol, organic acids and so forth may be suitably used. As the nitrogen source, ammonia gas, aqueous ammonia, ammonium salts and others may be used. As the inorganic ions, magnesium ions, phosphate ions, potassium ions, iron ions, manganese ions and others may suitably be used as required. As the organic nutrients, vitamins, amino acids, materials containing these substances such as lever extract, yeast extract, malt extract, peptone, meat extract, corn steep liquor, casein decomposition products and others may suitably be used.

Moreover, by adding saccharides and sugar alcohols such as D-xylose, D-xylulose, D-arabitol and xylitol to the medium as an inducer for the enzyme, the activity converting D-arabitol into D-xylulose may be improved.

The culture conditions are also not particularly limited, and culture may be performed, for example, at pH 5-8 and a temperature within the range of 25-40°C for 12 to 72 hours under an aerobic condition with suitable control of pH and temperature.

D-Xylulose can be produced in a reaction mixture by contacting a culture containing cells obtained as described above, cells separated and collected from the culture, processed cells subjected to acetone treatment or lyophillization, cell free extract prepared from such cells or processed cells, fractions such as membrane fractions fractionated from such cell free extract, or immobilized materials produced by immobilizing these cells, processed cells, cell free extract and fractions with D-arabitol, and allowing the reaction. The microorganism to be used may consist of one kind of microorganism, or may be used as an arbitrary mixture of two or more kinds of them.

In general, by performing the reaction at a temperature of 20-60°C, desirably 30-40°C, and at pH 4.0-9.0, desirably pH 6.5-8.0, a good result can be obtained. The reaction may be performed as a standing reaction or reaction with stirring. The reaction time may vary depending on various conditions such as activity of the microorganism to be used and D-arabitol concentration, but it is desirably 1-100 hours. Further, by adding a carbon source such as glucose and ethanol to the reaction, the production amount of D-xylulose may be improved. Moreover, by adding pyrroloquinoline quinone to the reaction mixture, the production amount of D-xylulose may be improved.

Further, by culturing the aforementioned microorganism in a medium containing D-arabitol, the reaction generating D-xylulose from D-arabitol can be attained in parallel to the culture.

When xylitol is produced by the method of the present invention, D-xylulose produced as described above may be used as the reaction mixture as it is after the reaction is completed, or used after collection from the mixture and purification. In order to collect and separate D-xylulose from a reaction mixture after the reaction is completed, methods using synthetic adsorptive resins, methods using precipitating agents and other usual collection and separation methods can be used.

Examples of the microorganism having an ability to produce D-xylulose from glucose include microorganisms belonging to the geneus *Gluconobacter*, *Acetobacter* or *Frateuria*.

Specific examples of such microorganisms include:
*Gluconobacter cerinus*, *Gluconobacter oxydans*, *Gluconobacter asaii*,
*Acetobacter aceti*, *Acetobacter liquefaciens*, *pasteurianus*,
*Frateuria auranti*a and so forth.

Specific bacterial strains include:
*Gluconobacter cerinus* IFO3262
*Gluconobacter oxydans* ATCC8147
*Gluconobacter oxydans* IFO3293
*Gluconobacter oxydans* IAM1839
*Gluconobacter oxydans* IFO3250
*Gluconobacter oxydans* IFO3292
*Gluconobacter oxydans* IFO3294
*Gluconobacter oxydans* subsp. oxydans IFO3189
*Gluconobacter oxydans* subsp. suboxydans IFO3172
*Gluconobacter oxydans* subsp. suboxydans IFO3130
*Acetobacter aceti* subsp. xylinum ATCC14851
*Acetobacter liquefaciens* ATCC14835
*Acetobacter pasteurianus* IFO3222
*Acetobacter pasteurianus* IFO3223
*Frateuria aurantia* IFO3245

*Gluconobacter oxydans* ATCC621 and *Gluconobacter* *oxydans* ATCC8147, *Acetobacter aceti* subsp. xylinum ATCC14851, and *Acetobacter liquefaciens* ATCC14835 are available from the American Type Culture Collection (Address: 12301 Parklawn Drive, Rockville, Maryland 20852, United States of America).

*Gluconobacter cerinus* IFO3262, *Gluconobacter oxydans* IFO3293, *Gluconobacter oxydans* IFO3250, *Gluconobacter oxydans* IFO3292, *Gluconobacter oxydans* IFO3294, *Gluconobacter oxydans* subsp. oxydans IFO3189, *Gluconobacter oxydans* subsp. suboxydans IFO3172, *Gluconobacter oxydans* subsp. suboxydans IFO3130, *Acetobacter pasteurianus* IFO3222, *Acetobacter pasteurianus* IFO3223 and *Frateuria aurantia* IFO3245 are available from the Institute for Fermentation, Osaka (Address: 17-85, Juso-honmachi 2-chome, Yodogawa-ku, Osaka 532, Japan).

*Gluconobacter oxydans* IAM1839 is available from the Institute of Molecular and Cellular Biosciences (Formerly, Institute of Applied Microbiology, The University of Tokyo, Address: Yayoi 1-chome, Bunkyo-ku, Tokyo, Japan.

The culture medium for culturing the aforementioned microorganisms may be a usual culture medium containing a usual carbon source, nitrogen source, inorganic ions, as well as organic nutrients as required.

As the carbon source, carbohydrates such as glucose, alcohols such as glycerol, organic acids and the like can suitably be used. In view of the preference observed in the known methods for the production of xylitol, for example, the method for producing xylitol from pentitols such as D-xylose or D-arabitol, preferred are hexoses such as glucose and fructose, disaccharides such as sucrose and lactose, and polysaccharides such as starch. These materials are used as a main carbon source in the medium in an amount of 10-60%, preferably 20-50%. These carbon sources may be added to the culture medium at one time, or divided into portions and added portionwise over the culture time course.

As the nitrogen source, ammonia gas, aqueous ammonia, ammonium salts and so forth are used. As the inorganic ions, magnesium ions, phosphate ions, potassium ions, iron ions, manganese ions and so forth are suitably used as required. As the organic nutrient, vitamins, amino acids, materials containing them such as lever extract, yeast extract, malt extract, peptone, meat extract, corn steep liquor, casein decomposition products and so forth are used as required.

The culture conditions are also not particularly limited. However, the microorganisms may be cultured, in general, within a pH range of 5-8 and temperature range of 25-40°C with suitable control of pH and temperature. The culture is performed under an aerobic condition obtained by, for example, stirring or shaking for aeration. As for the culture time, the microorganisms are desirably cultured until the main carbon source is consumed, i.e., usually for 3-8 days.

When xylitol is produced by the method of the present invention, D-xylulose produced in the medium as described above may be used as the medium as it is, or used after collection from the medium and purification. In order to collect and separate D-xylulose from the medium, methods using synthetic adsorptive resins, methods using precipitating agents and other usual collection and separation methods can be used.

Xylitol produced as described above can be separated and collected from the reaction mixture in a conventional manner. Specifically, for example, after the solid matter is removed from the reaction mixture by centrifugation, filtration or the like, the residual solution can be decolorized and desalted by using activated carbon, ion-exchange resin or the like, and xylitol can be crystallized from the solution.

According to the present invention, xylitol can efficiently be produced from glucose, D-arbitol or D-xylulose.

### Best Mode for Carrying out the Invention

The present invention will be explained more specifically with reference to the following examples. However, the present invention is not limited to these examples. In the examples, D-arabitol as the raw material, and the produced xylitol and D-xylulose were analyzed by high performance liquid chromatography (HPLC) under the following conditions.
- Column:: Shodex SC1211 (Showa Denko, Japan)
- Mobile phase:: 50% acetonitrile/50% 50 ppm aqueous solution of Ca-EDTA
- Flow rate:: 0.8 ml/minute
- Temperature:: 60°C
- Detection:: RI detector

### Example 1: Production of Escherichia coli transformed with gene encoding xylitol dehydrogenase

Based on the nucleotide sequence of the known gene encoding xylitol dehydrogenase of *Morganella morganii* ATCC25829 strain (DDBJ/GenBank/EMBL Accession No. L34345), the oligonucleotides having the nucleotide sequences represented as SEQ ID NO: 1 and 2 in Sequence Listing were synthesized.

PCR was performed with usual conditions using these synthetic oligonucleotides as primers and the genomic DNA of the strain as a template. As a result, a DNA fragment of about 1.2 kb containing the gene encoding xylitol dehydrogenase was amplified. The DNA fragment was separated and collected by agarose gel electrophoresis. Then, the both ends of the fragment were digested with *Eco*RI and *Bam*HI, and ligated to an *Eco*RI and *Bam*HI digestion product of pUC18 (Takara Shuzo). This was used for the transformation of *Escherichia coli* JM109, and the transformant strains containing the gene encoding xylitol dehydrogenase were selected. If a cloned fragment contains the gene encoding xylitol dehydrogenase, it is expressed under the control of *lac* promoter as a fusion gene with *lac Z*' gene.

The xylitol dehydrogenase activity of transformant strains was measured as follows. Cells cultured overnight in L medium (1% of trypton, 0.5% of yeast extract, 0.5% of NaCl) were sonicated, and the supernatant was used as a crude enzyme solution. The reaction was performed in 1 ml of a reaction mixture containing 100 mM glycine buffer (pH 9.5), 100 mM xylitol, 2 mM NAD and 100 µl of the crude enzyme solution at 30°C for 1 minute. The production of NADH or NADPH was detected based on the increase of absorbance at 340 nm. The enzymatic activity for oxidizing 1 µmol of xylitol to generate 1 µmol of NADH per minute under the aforementioned reaction condition was defined as one unit.

As a result, 3.9 U/mg of xylitol dehydrogenase activity was recognized in the transformant strain, whereas the enzyme activity was not observed in *Escherichia coli* JM109 (host strain) used as a control. Thus, it was confirmed that the transformant strain had the gene encoding xylitol dehydrogenase.

### Example 2: Production of xylitol from D-arabitol using Gluconobacter oxydans and Escherichia coli transformed with gene encoding xylitol dehydrogenase

### (1) Conversion of D-arabitol into D-xylulose

The *Gluconobacter oxydans* ATCC621 strain was inoculated into a test tube including 3 ml of a medium (pH 6.0) containing 2.4% of potato dextrose, 3% of yeast extract, 0.5% of glycerol, 3% of mannitol and 2% of calcium carbonate, and cultured at 30°C for 16 hours. Then, the above medium was inoculated in an amount of 5% to a 500-ml volume flask including 50 ml of a medium (pH 6.0) containing 0.5% of yeast extract (w/v), 0.5% of peptone, 0.5% of beef extract, 0.5% of ammonium sulfate, 0.1% of potassium dihydrogenphosphate, 0.3% of dipotassium hydrogenphosphate, 0.05% of magnesium sulfate heptahydrate, 0.001% of iron sulfate heptahydrate, 0.001% of manganese sulfate n-hydrate, 5% of D-arabitol and 2% of calcium carbonate, and cultured at 30°C with shaking. As a result, 4.7% (yield: 94%) of D-xylulose was obtained in 17 hours.

### (2) Production of xylitol from D-xylulose

The cells were removed by centrifugation from the above-obtained medium containing D-xylulose, and then the D-xylulose was converted into xylitol by using the *Escherichia coli* transformed with a gene encoding xylitol dehydrogenase produced in Example 1.

First, the aforementioned transformant strain was inoculated into a test tube including 4 ml of a medium (pH 6.0) containing 0.5% of yeast extract, 0.5% of peptone, 0.5% of beef extract, 0.5% of ammonium sulfate, 0.1% of potassium dihydrogenphosphate, 0.3% of dipotassium hydrogenphosphate, 0.05% of magnesium sulfate heptahydrate, 0.001% of iron sulfate heptahydrate, 0.001% of manganese sulfate n-hydrate, 1% of glycerol, 5% of D-arabitol and 2% of calcium carbonate, and cultured at 30°C for 16 hours with shaking. Then, 1 ml of the medium was inoculated to a 500-ml volume flask containing 50 ml of the same medium, and cultured for 3 hours. IPTG (isopropyl-β-D-thiogalactopyranoside) was added to the medium at a final concentration of 1 mM as an expression inducer of the lac promoter, and the culture was continued for 4 hours. The medium obtained as described above was added to the culture supernatant of *Gluconobacter oxydans* containing D-xylulose, which was obtained in (1), at a final concentration of 5%, and cultured at 30°C with shaking. As a result, 4.3% (yield: 91%) of xylitol was obtained in 40 hours.

Combining the processes of the above (1) and (2), xylitol at a concentration of 4.3% (yield: 86%) could efficiently be produced from D-arabitol at a concentration of 5% in 57 hours in total by using *Gluconobacter oxydans* and *Escherichia coli* transformed with a gene encoding xylitol dehydrogenase.

### Example 3: Production of xylitol from D-arabitol simultaneously using Gluconobacter oxydans and Escherichia coli transformed with gene encoding xylitol dehydrogenase

In the same manner as in Example 2, the *Gluconobacter oxydans* ATCC621 strain was inoculated into a test tube including 3 ml of a medium (pH 6.0) containing 2.4% of potato dextrose, 3% of yeast extract, 0.5% of glycerol, 3% of mannitol and 2% of calcium carbonate, and cultured at 30°C for 16 hours.

On the other hand, the *Escherichia coli* transformed with a gene encoding xylitol dehydrogenase, which was produced in Example 1, was similarly inoculated into a test tube including 4 ml of a medium (pH 6.0) containing 0.5% of yeast extract, 0.5% of peptone, 0.5% of beef extract, 0.5% of ammonium sulfate, 0.1% of potassium dihydrogenphosphate, 0.3% of dipotassium hydrogenphosphate, 0.05% of magnesium sulfate heptahydrate, 0.001% of iron sulfate heptahydrate, 0.001% of manganese sulfate n-hydrate, 5% of D-arabitol and 2% of calcium carbonate, and cultured at 30°C for 16 hours with shaking. Then, 1 ml of the medium was inoculated to a 500-ml volume flask containing 50 ml of the same medium, and cultured for 3 hours. IPTG was added to the medium at a final concentration of 1 mM as an expression inducer, and the culture was continued for 4 hours.

The co-cultivation medium of *Gluconobacter oxydans* and the *Escherichia coli* transformed with a gene encoding xylitol dehydrogenase was inoculated into a 500-ml volume flask including 50 ml of a medium (pH 6.0) containing 0.5% of yeast extract (w/v), 0.5% of peptone, 0.5% of beef extract, 0.5% of ammonium sulfate, 0.1% of potassium dihydrogenphosphate, 0.3% of dipotassium hydrogenphosphate, 0.05% of magnesium sulfate heptahydrate, 0.001% of iron sulfate heptahydrate, 0.001% of manganese sulfate n-hydrate, 5% of D-arabitol and 2% of calcium carbonate, and cultured at 30°C with shaking.

As a result, xylitol was obtained at a concentration of 4.4% (yield: 88%) from D-arabitol at a concentration of 5% in 50 hours.

### Reference Example 1: Production of D-xylulose from D-arabitol

To test tubes, 4 ml for each tube of a medium (pH 7.0) containing 1.8% (w/v) of nutrient broth (produced by EIKEN CHEMICAL CO., LTD.) was introduced, and sterilized by heating at 120°C for 20 minutes. D-Arabitol, xylitol and D-xylose separately sterilized were added to the medium each at a concentration of 1%. Cells for each of the strains shown in Table 1 were inoculated into the aforementioned medium, and cultured at 30°C for two days with shaking. The cells were collected from the medium by centrifugation, and washed once with physiological saline.

The cultured cells of each strain were suspended in 0.1 M phosphate buffer (pH 7.0) at a concentration of about 5% (w/v) in terms of wet weight. Glass beads (produced by Biospec Products, diameter: 0.1 mm) were added to the suspension in a volume of about a half of the cell suspension, and the cells were disrupted by using Multi-Beads Shocker MB-200 (Yasui Kikai). The disruption was performed at 3000 rpm for 3 minutes, and the obtained disrupted cell suspension was used as a crude enzyme preparation in the following conversion reaction.

D-Arabitol and NAD were dissolved in 1 M Tris-HCl buffer (pH 8.0) at final concentrations of 5% (w/v) and 10 mM, respectively, and introduced into test tubes in an amount of 0.9 ml for each tube. To this reaction mixture, 0.1 ml of each crude enzyme preparation was added, and allowed to react at pH 8.0 and 30°C for 22 hours. After the reaction, precipitates were removed by centrifugation, and the produced D-xylulose was measured by HPLC. The results are shown in Table 1. As shown in Table 1, D-xylulose was efficiently produced from D-arabitol and accumulated with cells of every strain.

**Table 1**

| Strain | Produced D-xylulose (g/l) | Reaction yield (%) |
|---|---|---|
| *Achromobacter delmarvae* AJ1983 | 0.4 | 0.8 |
| *Achromobacter viscosus* ATCC12448 | 0.1 | 0.2 |
| *Agrobacterium tumefaciens* ATCC4720 | 0.3 | 0.6 |
| *Agrobacterium radiobacter* ATCC4718 | 0.1 | 0.2 |
| *Alcaligenes faecalis* IAM1015 | 0.6 | 1.2 |
| *Arthrobacter citreus* ATCC11624 | 0.3 | 0.6 |
| *Arthrobacter tumescens* ATCC6947 | 0.4 | 0.8 |
| *Arthrobacter paraffineus* ATCC15590 | 0.7 | 1.4 |
| *Arthrobacter paraffineus* ATCC15591 | 1.3 | 2.6 |
| *Arthrobacter paraffineus* ATCC19064 | 0.8 | 1.6 |
| *Arthrobacter paraffineus* ATCC19065 | 1.0 | 2.0 |
| *Arthrobacter hydrocarboglutamicus* ATCC15583 | 0.9 | 1.8 |
| *Azotobacter indicus* ATCC9037 | 0.1 | 0.2 |
| *Brevibacterium ammoniagenes* ATCC6871 | 0.4 | 0.8 |
| *Brevibacterium ammoniagenes* ATCC6872 | 0.5 | 1.0 |
| *Brevibacterium divaricatum* ATCC14020 | 0.5 | 1.0 |
| *Brevibacterium lactofermentum* ATCC13655 | 0.5 | 1.0 |
| *Brevibacterium flavum* ATCC13826 | 1.3 | 2.6 |
| *Brevibacterium flavum* ATCC21406 | 0.3 | 0.6 |
| *Brevibacterium globosum* AJ1563 | 0.1 | 0.2 |
| *Brevibacterium fuscum* FERM BP-6983 | 0.5 | 1.0 |
| *Brevibacterium ketoglutamicum* ATCC15587 | 0.4 | 0.8 |
| *Brevibacterium ketoglutamicum* ATCC15588 | 0.9 | 1.8 |
| *Corynebacterium acetophilum* NRRLB3421 | 0.3 | 0.6 |
| *Enterobacter aerogenes* ATCC13048 | 0.1 | 0.2 |
| *Erwinia amylovora* IFO12687 | 0.1 | 0.2 |
| *Erwinia carotovora* CCM969 | 0.1 | 0.2 |
| *Flavobacterium peregrinum* CCM1080-A | 0.1 | 0.2 |
| *Flavobacterium fucatum* FERM BP-6492 | 0.1 | 0.2 |
| *Micrococcus* sp. CCM825 | 0.3 | 0.6 |
| *Nocardia opaca* NCIB9409 | 1.0 | 2.0 |
| *Planococcus eucinatus* FERM BP-6493 | 0.4 | 0.8 |
| *Proteus rettgeri* NRRL 11344 | 0.7 | 1.4 |
| *Proteus rettgeri* FERM BP-6980 | 0.7 | 1.4 |
| *Proteus rettgeri* FERM BP-941 | 0.6 | 1.2 |
| *Morganella morganii* AJ2771 | 0.5 | 1.0 |
| *Propionibacterium shermanii* IAM1725 | 0.1 | 0.2 |
| *Pseudomonas synxantha* ATCC796 | 0.4 | 0.8 |
| *Rhodococcus erythropolis* ATCC11048 | 1.1 | 2.2 |
| *Sparosarcina ureae* FERM BP-6979 | 0.1 | 0.2 |
| *Staphylococcus aureus* IFO3761 | 0.1 | 0.2 |
| *Vibrio metschnikovii* ATCC7708 | 0.1 | 0.2 |
| *Vibrio tyrogenes* FERM BP-5848 | 0.6 | 1.2 |

### Reference Example 2: Production of D-xylulose from D-arabitol

To 500-ml volume flasks, 50 ml for each flask of a medium (pH 7.2) containing 0.2% of yeast extract (w/v), 0.2% of meat extract, 0.4% of peptone, 0.5% of NaCl and 0.2% of magnesium sulfate heptahydrate was introduced, and sterilized by heating at 120°C for 20 minutes. D-Arabitol, xylitol and D-xylose separately sterilized were added to the medium each at a concentration of 1%. Cells for each of the strains shown in Table 1 were inoculated into the medium, and cultured at 30°C for two days with shaking. The cells were collected from the medium by centrifugation, and washed once with physiological saline.

The cultured cells of each strain were suspended in 0.1 M phosphate buffer (pH 7.0) at a concentration of about 5% (w/v) in terms of wet weight. Glass beads (produced by Biospec Products Co., diameter: 0.1 mm) were added to the suspension in a volume of about a half of the cell suspension, and the cells were disrupted by Multi-Beads Shocker MB-200 (Yasui Kikai). The disruption was performed at 3000 rpm for 3 minutes, and the obtained disrupted cell suspension was used as an a crude enzyme preparation in the following conversion reaction.

D-Arabitol and NAD were dissolved in 1 M Tris-HCl buffer (pH 8.0) at final concentrations of 5% (w/v) and 10 mM, respectively, and introduced into test tubes in an amount of 0.9 ml for each tube. To this reaction mixture, 0.1 ml of each crude enzyme preparation was added, and allowed to react at pH 8.0 and 30°C for 22 hours. After the reaction, precipitates were removed by centrifugation, and the produced D-xylulose was measured by HPLC. The results are shown in Table 2. As shown in Table 2, D-xylulose was efficiently produced from D-arabitol and accumulated with cells of every strain.

**Table 2**

| Strain | Produced D-xylulose (g/l) | Reaction yield (%) |
|---|---|---|
| *Actinomadura madurae* ATCC19425 | 0.1 | 0.2 |
| *Actinomyces violaceochromogenes* IFO13100 | 0.7 | 1.4 |
| *Kitasatosporia parulosa* AJ9458 | 0.1 | 0.2 |
| *Streptomyces antibioticus* NRRL3238 | 0.1 | 0.2 |
| *Streptomyces cacaoi* ATCC19093 | 0.1 | 0.2 |
| *Streptomyces coelicolor* ATCC10147 | 0.1 | 0.2 |
| *Streptomyces flavelus* AJ9012 | 0.1 | 0.2 |
| *Streptomyces griseolus* NRRL B-1062 | 0.1 | 0.2 |
| *Streptomyces lavendulae* ATCC8664 | 0.1 | 0.2 |
| *Streptomyces lividans* IFO13385 | 0.1 | 0.2 |
| *Streptomyces olivaceus* ATCC21379 | 0.1 | 0.2 |
| *Streptomyces tanashiensis* ATCC15238 | 0.1 | 0.2 |
| *Streptomyces virginiae* AJ9053 | 0.1 | 0.2 |
| *Streptomyces viridochromogenes* IFO3113 | 0.1 | 0.2 |

### Reference Example 3: Production of D-xylulose from glucose

To test tubes, 4 ml for each tube of a medium containing 0.2% of ammonium sulfate, 0.1% of potassium dihydrogenphosphate, 0.3% of dipotassium hydrogenphosphate, 0.05% of magnesium sulfate and 0.5% of yeast extract (pH 6.0) was introduced, and sterilized by heating at 120°C for 20 minutes. D-Glucose separately sterilized was added to the medium at a concentration of 5%. The concentrations (%) were represented in weight/volume (w/v) percent.

Each of the strains shown in Table 3 was inoculated into the aforementioned medium, and cultured at 30°C for one day with shaking. This was used as seed culture. To 500-ml volume Sakaguchi flasks, 50 ml for each flask of a medium having the same composition as mentioned above (except for D-glucose) was introduced, and sterilized by heating at 120°C for 20 minutes. D-Glucose and calcium carbonate separately sterilized were added to the medium at concentrations of 5% and 4%, respectively. The aforementioned seed culture was inoculated into the medium at a concentration of 2%, and cultured at 30°C for 4 days with shaking. The cells were removed from the medium by centrifugation, and xylitol and D-xylulose produced in the medium were measured by HPLC. The results are shown in Table 3.

**Table 3**

| Strain | Produced D-xylulose (g/l) |
|---|---|
| *Gluconobacter cerinus* IFO3262 | 1.11 |
| *Gluconobacter oxydans* ATCC8147 | 0.4 |
| *Gluconobacter oxydans* IFO3293 | 1.0 |
| *Gluconobacter oxydans* IAM1839 | 0.5 |
| *Gluconobacter oxydans* IFO3250 | 0.5 |
| *Gluconobacter oxydans* IFO3292 | 1.0 |
| *Gluconobacter oxydans* IFO3294 | 1.0 |
| *Gluconobacter oxydans* subsp. oxydans IFO3189 | 1.0 |
| *Gluconobacter oxydans* subsp. suboxydans IFO3172 | 0.5 |
| *Gluconobacter oxydans* subsp. suboxydans IFO3130 | 1.2 |
| *Acetobacter aceti* subsp. xylinum ATCC14851 | 1.6 |
| *Acetobacter liquefaciens* ATCC14835 | 1.1 |
| *Acetobacter pasteurianus* IFO3222 | 1.0 |
| *Acetobacter pasteurianus* IFO3223 | 2.3 |
| *Frateuria aurantia* IFO3245 | 0.8 |

## Claims

1. A method for producing xylitol comprising the steps of reacting a microorganism which is transformed by a gene encoding xylitol dehydrogenase and has an ability to supply reducing power with D-xylulose to produce xylitol, and collecting the produced xylitol.

2. The method according to claim 1, wherein the microorganism having an ability to supply reducing power is a bacterium belonging to the genus *Escherichia*.

3. The method according to claim 2, wherein the bacterium belonging to the genus *Escherichia* is *Escherichia coli*.

4. The method according to claim 1, comprising the steps of reacting a microorganism which has an ability to convert D-arabitol into D-xylulose with D-arabitol to produce D-xylulose, and reacting the produced D-xylulose with a microorganism which is transformed by a gene encoding xylitol dehydrogenase and has an ability to supply reducing power.

5. The method according to claim 4, wherein the microorganism which has an ability to convert D-arabitol into D-xylulose is a microorganism belonging to the genus *Gluconobacter*, *Achromobacter*, *Agrobacterium*, *Alcaligenes*, *Arthrobacter*, *Azotobacter*, *Brevibacterium*, *Corynebacterium*, *Enterobacter*, *Erwinia*, *Flavobacterium*, *Micrococcus*, *Morganella*, *Nocardia*, *Planococcus*, *Proteus*, *Propionibacterium*, *Pseudomonas*, *Rhodococcus*, *Sporosarcina*, *Staphylococcus*, *Vibrio*, *Actinomadura*, *Actinomyces*, *Kitasatosporia*, *Streptomyces*, *Aeromonas*, *Aureobacterium*, *Bacillus*, *Escherichia*, *Microbacterium*, *Serratia*, *Salmonella* or *Xanthomonas*.

6. The method according to claim 1, which comprises steps of culturing a microorganism which has an ability to produce D-xylulose from glucose in a suitable medium to produce D-xylulose, and reacting the produced D-xylulose produced with a microorganism which is transformed by a gene encoding xylitol dehydrogenase and has an ability to supply reducing power in the medium.

7. The method according to claim 6, wherein the microorganism which has an ability to produce D-xylulose from glucose is a microorganism belonging to the genus *Gluconobacter*, *Acetobacter* or *Frateuria*.

8. A method for producing xylitol comprising steps of culturing a microorganism which is transformed by a gene encoding xylitol dehydrogenase and has an ability to supply reducing power and a microorganism which has an ability to convert D-arabitol into D-xylulose in a medium containing D-arabitol to produce xylitol, and collecting the produced xylitol.

9. A method for producing xylitol comprising steps of culturing a microorganism which is transformed by a gene encoding xylitol dehydrogenase and has an ability to supply reducing power and a microorganism which has an ability to produce D-xylulose from glucose in a suitable medium to produce xylitol, and collecting the produced xylitol.

10. A microorganism which is transformed by a gene encoding xylitol dehydrogenase and has the ability to supply reducing power to its culture medium.
